(19)

**Europäisches Patentamt**

**European Patent Office**

**Office européen des brevets**

(11) **EP 4 138 003 A1**

(12) **EUROPEAN PATENT APPLICATION**

(43) Date of publication:
**22.02.2023 Bulletin 2023/08**

(21) Application number: **21306133.6**

(22) Date of filing: **20.08.2021**

(51) International Patent Classification (IPC):
**G06N 20/00** (2019.01)        **G16B 20/20** (2019.01)
**G16B 30/10** (2019.01)

(52) Cooperative Patent Classification (CPC):
**G06N 20/00; G16B 20/20; G16B 30/10**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Dassault Systèmes
78140 Vélizy-Villacoublay (FR)**

(72) Inventors:
• **BALL, Arthur
  Vélizy-Villacoublay (FR)**
• **PECUCHET, Nicolas
  Vélizy-Villacoublay (FR)**

(74) Representative: **Bandpay & Greuter
30, rue Notre-Dame des Victoires
75002 Paris (FR)**

(54) **NEURAL NETWORK FOR VARIANT CALLING**

(57)    The disclosure notably relates to a computer-implemented method for machine-learning a neural network for variant calling with respect to a reference genome. The neural network is configured to take as input one or more sets of data pieces each specifying a respective read aligned relative to a genomic position of the reference genome. The neural network is configured to output information with respect to presence of a variant at the genomic position. The neural network comprises, for each set of data pieces, a respective function configured to take as input and process the set of data pieces. The respective function is symmetric. The machine-learning method improves variant calling with respect to a reference genome.

EP 4 138 003 A1

**Description**

**TECHNICAL FIELD**

The disclosure relates to the field of computer programs and systems, and more specifically to variant calling with respect to a reference genome.

**BACKGROUND**

In the last decades, genome sequencing has spread thanks to the technological progress achieved by New Genomic Sequencing (NGS) platforms. As genomes are made available, medical applications are being developed to take advantage of that. For example, personalized and targeted medicine have been getting more attention lately. Direct applications comprise, e.g., genetic disorders diagnosis and cancer profiling for treatment.

Genome sequencing is a field that keeps evolving rapidly, both on the hardware front and on the software front, to respond to the growing need of precision medicine and epidemiology. In general, sequencing is often used to determine genomic variants present in a single individual. Variants are identified (i.e., "called") based on a comparison against a standard genome reference (Genome Reference Consortium). Variants are of different kinds: punctual variants that substitute a base in the reference are named "SNP" (Single Nucleotide Polymorphism), variants that remove bases compared to the reference are named "deletions", and variants that increase them (i.e., add one or more bases to the reference) are named "insertions". Finally, variants spanning a large number of bases are named "structural variants".

The process of variant calling may comprise an examination of each genomic position in order to determine which alleles are present in the diploid germinal genome: (i) homozygous if both alleles are identical, or (ii) heterozygous if alleles are different. Each allele can match the reference genome or otherwise be a variant. For the tumoral case, a variant is called if the genomic position does not match the reference or any of the germline alleles located at the same position.

The native DNA is fragmented and sequenced directly or after a pre-amplification step, so as to produce reads. Reads length varies from 100bp (e.g., using the Illumina technology) to 10kbp (e.g., using the Nanopore technology). One of the common characteristics of those technologies is the use of redundant sequencing to counterbalance experimental errors and to account for genetic heterogeneity in the cell population. The average number of reads per position is named the "depth" of the sequencing.

Variant calling is an active area of research since it is critical to have accurate and performant algorithms to develop genomics at a population scale. Performance has improved and is likely to keep improving in the near future. In particular, lately, bioinformatics researchers have tried to take advantage of machine-learning methods. This is notably the case of Deepvariant developed by Google. One thing to note is that the problem is quite difficult for normal DNA but it is even more challenging for tumoral DNA. Indeed, tumor cells mutate extremely rapidly, and thus they do not share the same DNA: it is a heterogeneous population.

Within this context, there is still a need for an improved solution for variant calling with respect to a reference genome.

**SUMMARY**

It is therefore provided a computer-implemented method for machine-learning a neural network for variant calling with respect to a reference genome. The neural network is configured to take as input one or more sets of data pieces each specifying a respective read aligned relative to a genomic position of the reference genome. The neural network is configured to output information with respect to presence of a variant at the genomic position. The neural network comprises, for each set of data pieces, a respective function configured to take as input and process the set of data pieces. The respective function is symmetric.

The machine-learning method may comprise one or more of the following:

- each data piece includes a respective sequence of base descriptors of the respective read;
- each respective function comprises one or more convolutional layers for processing each respective sequence of base descriptors;
- each convolutional layer applies one or more one-dimensional convolutional filters;
- the base descriptors comprise one or more descriptors representing insertion size and/or deletion size, for example an insertion size descriptor and a deletion size descriptor;
- each respective function comprises a reduction layer for collapsing order-independently the output of the one or more convolutional layers into a set of features;
- the reduction layer further takes as input read descriptors for each respective read;

- the read descriptors comprise a haplotype support descriptor;
- the reduction layer comprises one or more order-independent operators, such as a mean and/or a standard deviation;
- the neural network comprises one or more fully connected layers configured for processing the output of each reduction layer and performing classification;
- the neural network further takes as input pile descriptors;
- the pile descriptors comprise a descriptor representing depth and/or a descriptor representing a Bayesian variant estimation; and/or
- the one or more sets of reads comprise a first set of reads for germline variants and a second set of reads for somatic variants, the neural network comprising:

  ○ a first function for the first set of reads and a second function for the second sets of reads, and
  ○ a layer for aggregating the output of the first and second functions.

It is further provided a first computer-implemented method for variant calling with respect to a reference genome. The first variant-calling method comprises providing, as input, one or more sets of data pieces each specifying a respective read aligned relative to a genomic position of the reference genome. The first variant-calling method comprises applying, to the input, a neural network machine-learnt according to the machine-learning method, so as to output information with respect to presence of a variant at the genomic position. The application of the neural network comprises applying, for each set of data pieces, the respective symmetric function of the neural network configured to take as input and process the set of data pieces.

It is further provided a second computer-implemented method for variant calling with respect to a reference genome which includes the first variant-calling method. The second variant-calling method comprises providing one or more sets of reads aligned relative to the reference genome. The second variant-calling method comprises determining a set of regions of interest in the reference genome by comparing the one or more sets of reads with the reference genome. For each given region of interest, the second variant-calling method comprises the following steps. A first step is to perform a haplotype reconstruction based on the provided one or more sets of reads of the given region to determine two or more haplotypes. A second step is to re-align the one or more sets of reads of the given region based on the two or more haplotypes. A third step is to deduce potential variants of the given region based on the re-aligned one or more sets of reads and the two or more haplotypes. A fourth step is to perform a coarse grain filtering to detect candidate variants from the potential variants. Each detected candidate variant corresponds to a respective genomic position. In a fifth step, for each detected candidate, the second variant-calling method comprises the following sub-steps. A first sub-step is to determine one or more sets of data pieces each specifying a respective read aligned relative to the genomic position corresponding to the detected candidate. A second sub-step is to perform the providing and the applying of the first variant-calling method with the one or more sets of data pieces.

The second variant-calling method may comprise one or more of the following:

- the performing of the haplotype reconstruction comprises:

  ○ inferring a set of potential haplotypes by enumerating a predetermined number of longest paths in a directed acyclic graph; and optionally
  ○ selecting a subset of haplotypes from the set of potential haplotypes, the subset of haplotypes being potential haplotypes of the set having the highest number of supporting reads, the subset of haplotypes corresponding to the two or more haplotypes that the haplotype reconstruction determines;

- the one or more sets of reads comprises a first set of reads for germline variants, the deducing of potential variants of the given region comprising, for germline variants, evaluating a probability that a variant is more probable than the reference;
- the one or more sets of reads comprises a second set of reads for somatic variants, the deducing of potential variants of the given region considering, for somatic variants:

  ○ a presence of a germline variant and/or the presence of a somatic variant, and
  ○ a somatic variant frequency;

It is further provided a data structure comprising a neural network machine-learnt according to the machine-learning method (i.e., data representing the neural network).

It is further provided a computer program comprising instructions for performing the machine-learning method.

It is further provided a computer program comprising instructions for performing the first variant-calling method and/or the second variant-calling method.

It is further provided a device comprising a data storage medium having recorded thereon the data structure and/or any or both the computer programs.

The device may form or serve as a non-transitory computer-readable medium, for example on a SaaS (Software as a service) or other server, or a cloud based platform, or the like. The device may alternatively comprise a processor coupled to the data storage medium. The device may thus form a computer system in whole or in part (e.g. the device is a subsystem of the overall system). The system may further comprise a graphical user interface coupled to the processor.

**BRIEF DESCRIPTION OF THE DRAWINGS**

Non-limiting examples will now be described in reference to the accompanying drawings, where:

- FIG. 1 shows a flowchart of the first variant-calling method;
- FIG. 2 shows a flowchart of the second variant-calling method;
- FIG. 3 shows an example of a variant calling process;
- FIG. 4 shows an example of haplotype reconstruction;
- FIG. 5 shows an example of a germline candidate variants count as a function of recall;
- FIG. 6 shows an example of a somatic candidate variants count as a function of recall;
- FIG. 7 shows an example of pile-up images;
- FIG. 8 shows an example of architecture of the neural network;
- FIG. 9 shows an example of architecture of the neural network for calling somatic variants;
- FIG. 10 shows an example of results for the precision, the recall and the F1-scores metrics for the germline case;
- FIG. 11 shows an example of results for the number of errors metric for the germline case;
- FIG. 12 shows an example of a comparison of results with prior-art variant callers for the somatic case; and
- FIG. 13 shows an example of the system.

**DETAILED DESCRIPTION**

It is proposed a computer-implemented method for machine-learning a neural network for variant calling with respect to a reference genome. The neural network is configured to take as input one or more sets of data pieces each specifying a respective read aligned relative to a genomic position of the reference genome. The neural network is configured to output information with respect to presence of a variant at the genomic position. The neural network comprises, for each set of data pieces, a respective function configured to take as input and process the set of data pieces. The respective function is symmetric.

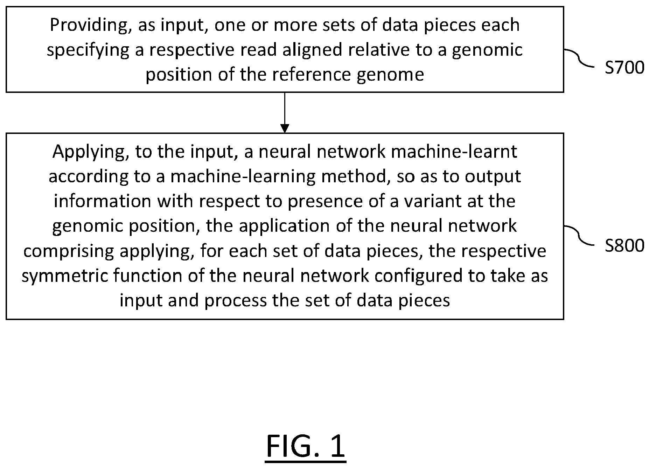

With reference to the flowchart of FIG. 1, it is further provided a first computer-implemented method for variant calling with respect to a reference genome. The first variant-calling method comprises providing S700, as input, one or more sets of data pieces each specifying a respective read aligned relative to a genomic position of the reference genome. The first variant-calling method comprises applying S800, to the input, a neural network machine-learnt according to the machine-learning method.

The neural network is configured to take as input the one or more sets of data pieces and to output information with respect to presence of a variant at the genomic position. The neural network comprises, for each set of data pieces, a respective function configured to take as input and process the set of data pieces. The respective function of each set is symmetric. The application of the neural network comprises applying, for each set of data pieces, the respective symmetric function of the neural network configured to take as input and process the set of data pieces.

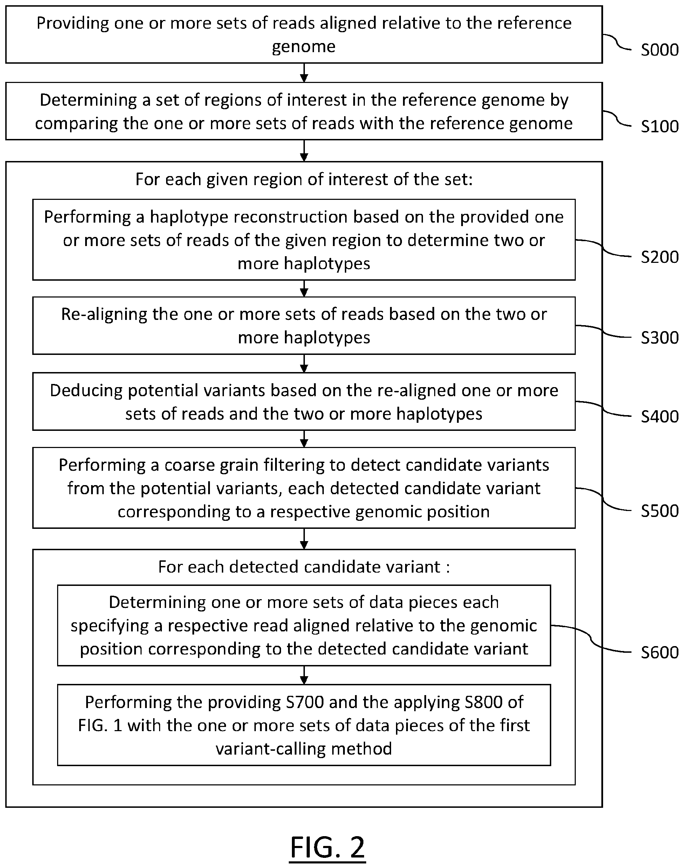

With reference to the flowchart of FIG. 2, it is further provided a second computer-implemented method for variant calling with respect to a reference genome, which incorporates the method of FIG. 1. The second variant-calling method comprises providing S000 one or more sets of reads aligned relative to the reference genome. The second variant-calling method comprises determining S100 a set of regions of interest in the reference genome by comparing the one or more sets of reads with the reference genome. For each given region of interest of the set, the second variant-calling method comprises the following steps. A first step consists in performing S200 a haplotype reconstruction based on the provided one or more sets of reads of the given region to determine two or more haplotypes. A second step consists in re-aligning S300 the one or more sets of reads of the given region based on the two or more haplotypes. A third step consists in deducing S400 potential variants of the given region based on the re-aligned one or more sets of reads and the two or more haplotypes. A fourth step consists in performing S500 a coarse grain filtering to detect candidate variants from the potential variants. Each detected candidate variant corresponds to a respective genomic position. In a fifth step, for each detected candidate, the second variant-calling method comprises the following sub-steps. A first sub-step comprises determining S600 one or more sets of data pieces each specifying a respective read aligned relative to the genomic position corresponding to the detected candidate. A second sub-step comprises performing the providing S700 and the applying S800 with the one or more sets of data pieces of the first variant-calling method.

The determining S100 of the set of regions may comprise executing a process which comprises going through the positions of the reference genome, and, for each given position computing a rate of mismatch in the reads aligned on the given position. During this step, the process may comprise recording (i.e. marking) positions having a rate that is higher than a predetermined threshold. At the same time, the process may aggregate them into regions of interest (those where the mismatch rate is several times higher than the threshold). As soon as the process computes a position with a rate higher than the threshold, the process may record the region as a region of interest. The determining S100 may comprise enlarging the region from one base to the next, within a predetermined limit (e.g., a limit of 1000 bases).

Such methods form improved solutions for variant calling with respect to the reference genome.

Notably, the machine-learning method takes advantage of machine learning techniques methods, such as speed, accuracy, and/or leveraging on available data. Furthermore, the machine-learning method allows an efficient and/or accurate machine-learning. Indeed, the respective function is symmetric, which allows the neural network learning to focus on essential information, as the order of the reads has no meaning in the sequencing output.

"Symmetric" refers to the mathematical definition of symmetric functions. In other words, a "symmetric" function is a function of several variables which is invariant to any permutation of its variables (whichever $\sigma$ any permutation, $f(x_1, ..., x_n) = f(\sigma(x_1, ..., x_n))$). Thus, the order of the read-specifying data pieces in each of the inputted one or more set has no influence on the result, i.e., on the information with respect to presence of the variant outputted by the neural network. In other words, the neural network is by construction invariant to the order of the read-specifying data pieces in each inputted set.

In case the neural network is configured to take as input several sets of data pieces and thus several functions, the functions may be the same, i.e. with the same parameters values and/or with shared weights. Alternatively, the functions may be distinct/different.

The machine-learning method may comprise providing a dataset of training samples for the reference genome, and training the neural network based on the provided dataset of training samples. Each training sample may comprise one or more sets of data pieces each specifying a respective read aligned relative to a given genomic position of the reference genome, and a corresponding output for the neural network, that is, output information with respect to presence of a variant at the given genomic position. Such output (i.e., ground truth) may be obtained in the construction of the dataset via traditional deterministic techniques. As known per se from the field of machine-learning, the processing of an input by a neural network includes applying operations to the input, the operations being defined by data including weight values. Learning/training a neural network thus includes determining values of the weights based on a dataset configured for such learning, such a dataset being possibly referred to as a learning dataset or a training dataset. The dataset may comprise training samples. Each training sample may comprise respective one or more sets of data pieces of respective reads aligned relative to a genomic position and respective information with respect to presence of a variant at the genomic position. The respective information may be the result of one or more preliminary studies and may be considered as ground truth for the learning. The training samples represent the diversity of the situations where the neural network is to be used after being learnt. The dataset may comprise a number of training samples higher than 1000, 10000, 100000, or 1000000. In the context of the present disclosure, by "learning a neural network", it is meant that the dataset is a learning/training dataset of the neural network, based on which the values of the weights (also referred to as "parameters").

The neural network may consist of a Deep Neural Network (DNN) and/or comprise several sub-networks. The machine-learning method may comprise training the several sub-networks conjunctly or separately. By "conjunctly" it is meant that the weights of the several sub-networks are all variables in a same single optimization. In case of several sets of data pieces, each function may form a respective sub-network, and the machine-learning method may comprise training said functions or sub-network respective to the several sets of data pieces conjunctly or separately.

The input of the neural network is now discussed.

A read is a sequence of bases (or equivalently a sequence of base pairs) of a respective DNA fragment. Any read herein may be obtained by DNA sequencing one or more cells based on any technology of DNA sequencing, such as Illumina technology, Ion Torrent technology or Nanopore technology. The sequencing may comprise fragmenting DNA of the one or more cells (i.e., native DNA) into a plurality of DNA fragments, and producing the reads based on the plurality of DNA fragments, optionally after a DNA amplification step. The one or more cells of any DNA sequencing herein may be sampled on an individual. Thus, the variant calling methods may each comprise initial steps of sampling one or more cells on an individual, DNA sequencing the cells as above so as to produce reads and then data pieces to be inputted to the neural network. The sampling may be performed in any manner and/or using any sampling instrument to take samples from an individual.

The reference genome may be a reference genome of any (single) species, in particular the human species. Any read herein may be a read of an individual of said species. In other words, the cell at the origin of the read may be a cell of said individual. The reference genome may consist of a digital nucleic acid sequence database representative of an example of the set of genes in one idealized individual organism of the species.

Each read is aligned relative to a genomic position, which means that the data piece specifying the read contains an indication of which portion the reference genome the read corresponds to or is mapped onto.

Each data piece forms a computer specification of a respective read aligned relative to a genomic position of the reference genome. In other words, each data piece comprises information that describes or relates to the respective read, for example the sequence of bases forming the read, and optionally additional information relating to the read, or to the bases therein. For instance, the information may comprise an encoded version of the sequence of bases of a respective read.

The output of the neural network is now discussed.

The outputted information may comprise an indication of whether or not a variant is present (i.e. at the genomic position), and optionally of the type of the variant (i.e., among a predetermined set of variant types). Additionally or alternatively, the outputted information may comprise a probability that a variant is present. The neural network may compute the probability that a variant is present by considering all the data pieces of each set. Alternatively, the neural network may output only significant probability of variant presence. For instance, the neural network may compute a probability of presence of a variant at the genomic position and output this computed probability only if the probability is higher than a threshold. The outputted information may also comprise other information such as information on the genomic position, information on a type of the variant, information on whether the variant is homozygote or heterozygote.

The predetermined set of types of variant may comprise one or more types for punctual variants (i.e., SNP), one or more types for deletions (i.e., variants that remove one or more bases compared to the reference), and one or more types for insertions (i.e., variants that increase one or more bases compared to the reference). For deletions and insertions, the number of removed or increased bases may have a maximum number. In other words, the neural network may output that a variant is an insertion respectively a deletion if the number of added respectively removed bases is less than or equal to the maximum number. The information may be outputted in any format, for example a text format or a table format, e.g. with one line or row per position determined to have a variant or to likely have a variant.

The neural network may be trained and designed to be applied to data pieces all specifying germline reads (i.e. reads from healthy cells) of an individual (e.g. of the human species). In this case, the output may indicate whether or not a variant is present in healthy cells of the individual. The neural network may be alternatively trained and designed to be applied to data pieces specifying somatic reads of an individual having a cancer disease (i.e. reads from cancer cells), and optionally together with data pieces specifying germline reads of the same individual as well. In that case, the output may indicate whether or not a variant is present in the somatic cells of the individual. This latter case allows to call variants specific to certain cancer diseases.

Applications of the variant calling methods are now discussed.

For the germline case, an application may be the prognosis, the diagnosis or the treatment of diseases (e.g. diagnosis of a genetic disease). For instance, the application may comprise analyzing the called variants of an individual, and diagnosing a disease and/or determining an adapted treatment (e.g. a dosage) for treating the diagnosed disease, and/or performing a prognosis and/or determining an adapted behavior. The individual (e.g. human) subject to the variant calling method may then follow such treatment and/or behavior.

For the somatic case, an application may be the evaluation or prognosis and the treatment of a cancer disease affecting a subject individual (e.g. determining details on the cancer, or confirming cancer diagnosis). The application may comprise identifying mutations in the somatic cells by comparing the variants called for the healthy cells with the variants called for the somatic cells. The application may further comprise providing a specific treatment for this cancer based on the called variants of the individual and the identified mutations. Alternatively or additionally, the application may comprise predicting an evolution of the cancer based on the identified mutations.

Any of the variant calling methods may comprise displaying output of the neural network, and/or a result of the above applications.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

Each data piece may include a respective sequence of base descriptors of the respective read. In other words, each data piece may comprise a vector of base descriptors having coordinates each corresponding to a respective position of the reference genome, and each vector has one coordinate corresponding to the aligned genomic position. The base descriptors may comprise a type of base (for example A, T, C or G) and the respective sequence of base descriptors may comprise the sequence of bases of the read. The length of each data piece may thus correspond to the size of the sequence of bases of the read (i.e., be variable depending on the size of each read). Alternatively, the length of each data piece may be homogeneous (e.g., constant) and the data pieces may be all centered on the genomic position. In that case, each data piece may further include at least one additional start sequence or end sequence positioned at the beginning or at the end of the sequence of base descriptors corresponding to the read and having a size that variates to align the respective sequence of each read on the reference genome. Each additional start or end sequence may consist of a sequence of one or more base descriptors having null values. In other words, the vectors are normalized by adding null values at the beginning and/or at the end of each vector, such that all vectors have the same length and all vectors have the same coordinate aligned on the genomic position. Such normalization facilitates generic processing of each set of base descriptor sequences, for example by convolutional layers.

Each respective function may comprise one or more convolutional layers for processing each respective sequence of base descriptors, i.e. each respective function applies one or more convolutions to each respective sequence of base descriptors. In case the neural network is configured to take as input several sets and thus comprises several symmetric functions, the one or more convolutional layers may be the same for the different functions, i.e. may comprise shared weights. Alternatively, the one or more convolutional layers may be distinct for the different functions. Each convolutional layer may apply one or more one-dimensional convolutional filters. Each one-dimensional convolution filter may take as input and process a vector $l$ x 1 representing each data piece, wherein $l$ is the length of each data piece. The application of one-dimensional convolutional filters allows considering each read independently and without considering the order of the reads. The efficiency and/or accuracy of the learning is thus increased. Indeed, the learning of the neural network focuses on essential information (by not trying to learn something from the order between the reads of the input, where there is nothing to learn there).

Each respective function may comprise a reduction layer for collapsing order-independently the output of the one or more convolutional layers into a set of features. For instance, the reduction layer may comprise one or more order-independent operators, such as a mean and/or a standard deviation. In the case of several sets and thus several symmetric functions, the reduction layer may be the same for the different functions, i.e. may comprise same parameters values (e.g. same order-independent operators). Alternatively, the reduction layer may be distinct for the different functions.

With reference to FIG.s 3 to 11 and in the following sections 1 to 3, a specific implementation of the machine-learning method and of the first and second variant-calling methods is now discussed.

Section 1: Variant calling process

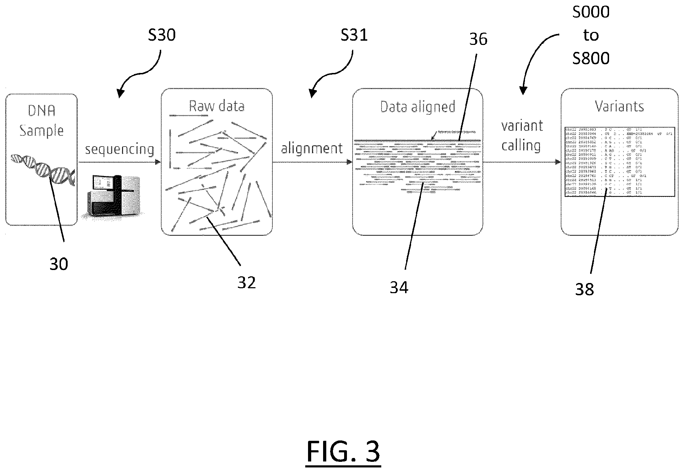

FIG. 3 illustrates an example of a variant calling process in which the specific implementation of the variant-calling method is implemented. The variant calling process comprises a sequencing S30 of a native DNA 30 of one individual (e.g. of the human species, such as a patient). The native DNA 30 may be fragmented and sequenced S30 directly or after a pre-amplification step to produce reads 32. The sequencing of the native DNA may be performed with any technology of DNA sequencing, such as the Illumina technology or the Nanopore technology. The reads length may vary depending on the technology of DNA sequencing. The reads length may vary from 100 bp (acronym for base pair) with the Illumina technology to 10 kbp (1 kbp = 1000 bp) with the Nanopore technology. One of the common characteristics of sequencing technologies that the method may implement is the use of redundant sequencing to counter-balance the experimental errors and account for genetic heterogeneity in the cell population. The average number of reads per position is called the depth of sequencing.

After the sequencing S30, the variant calling process comprises an alignment S31 of the reads 32 on the reference genome 36. The alignment S31 may consist of a mapping of the reads against the reference genome, for example based on a known mapping algorithm such as the Burrows-Wheeler Aligner. The alignment S31 may comprise a positioning of each read with the reference genome based on a sequence of bases similarity. The alignment S31 allows identifying similarities between the sequence of bases of each read and a respective sequence of bases of the reference genome. Due to the redundancy, several reads are aligned on each genomic position of the reference genome, and the several reads that align on a given genomic position form a pile of reads for the genomic position. The reads aligned relative to the reference genome may form a first set of reads.

In the variant calling process, the sequencing S30 and the alignment S31 steps may be repeated, successively or in parallel, for different samples of DNA. For instance, the sequencing S30 and the alignment S31 steps may be performed for a first sample for germline variants and for a second sample for somatic variants. In that case, the process forms several (e.g. two) sets of reads aligned relative to a genomic position. The formed one or more sets of reads are provided as input in the second variant calling method.

The output of the second variant-calling method is a list of variants 38, which results from a compilation of the information outputted by the neural network when applied to the provided one or more sets of reads for each detected candidate variant. The compilation may comprise a selection, from the information outputted for each detected candidate variant, of the list of variants 38 based on the probability of each variant. For each variant of the list, the output comprises information that describes the variant, for example information about the genomic position of the variant, information on the type of the variant and information on whether the variant is homozygote or heterozygote.

**Section 2: Variant Candidates detection**

2.1 Haplotype reconstruction and local realignment

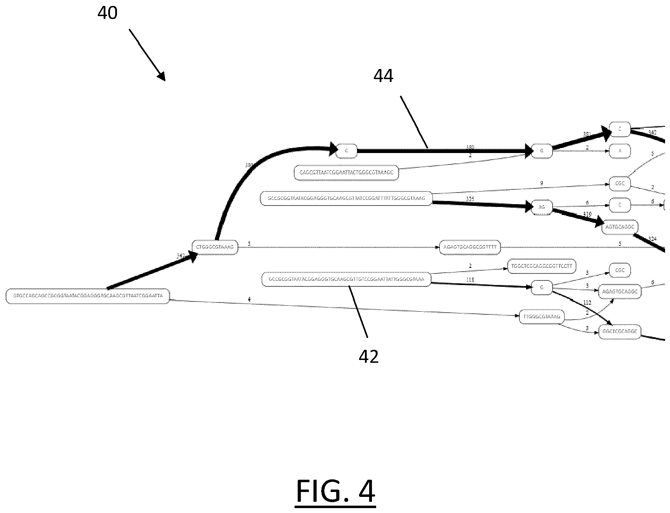

The steps of haplotype reconstruction and local realignment improve the recognition of variants. Indeed, the reconstruction of haplotypes allow combining information of all the reads that map to a same region, and the reconstructed haplotypes are then used for re-aligning the reads, which improves the alignment of the reads with the reference genome.
In order to save computing resources, the variant-calling method restricts it to the regions containing reads with numerous mismatches (i.e., the method restricts the haplotype reconstruction to a set of regions of interest). Indeed, the regions for which there are many differences with the reference genome are the regions in which variants can be found. The variant-calling method reconstructs haplotypes (i.e., performs local chromosome segments) in these regions of interest which allows realigning the reads correctly.
The determining the set of regions of interest may comprise determining positions with a mismatch rate between the reads and the reference genome above a given threshold and aggregating the determined positions into a set of regions of interest. For instance, the determining of positions with a mismatch rate above a given threshold may comprise successively going through positions of the reference genome (or of a portion of the reference genome) and, for each given position, computing a rate of difference in the reads aligned on this given position (i.e., an error rate). While going through the genome positions, the method may record a given position if the computed rate is above the threshold. Successively or at the same time, the method may aggregate the recorded positions into regions of interest. The aggregation may comprise selecting reads mapping to neighbor positions of the recorded positions, for example positions in a neighborhood defined by a predetermined number of bases on each side around a position (e.g., 100 bp on each side).
With reference to FIG. 4, an example of the haplotype reconstruction is now discussed. The haplotype reconstruction of the example is based on an acyclic De-Bruijn graph 40. The haplotype reconstruction of the example comprises splitting up each read into k-mers having an initial k-mer size and computing an initial De-Bruijn graph based on the k-mers. In the De-Bruijn graph, nodes 42 are k-mers and edges 44 indicate a transition between two consecutive k-mers. The initial k-mer size may variate between 20 and 30 bp to ensure minimal specificity. Indeed, on the one hand, the smaller the k-mers are, the less specific they are (and therefore the more likely it is that two k-mers that are located at different places may collide), and, on the other hand, small k-mers are less likely to contain experimental errors. An initial k-mer size between 20 and 30 bp is therefore a compromise that takes into account these two tendences. Then, the haplotype reconstruction comprise increasing the k-mer size until the De-Bruijn graph obtained is acyclic. The increasing of the k-mer size may be incremental, and a new De-Bruijn graph may be computed at each new k-mer size.
Potential haplotypes are then inferred by enumerating N longest paths (e.g., N = 100) in the computed acyclic De-Bruijn graph. An haplotype is a sequence of bases that includes the variants (haplotypes are determined statistically). It corresponds to the reference for a given patient. Then, the method selects a subset of haplotypes from the set of potential haplotypes. The haplotypes of the subset are potential haplotypes having the highest number of supporting reads. The subset of haplotypes corresponds to the two or more haplotypes that the haplotype reconstruction finally determines. To select the subset of haplotypes, the method enumerates haplotypes by filtering relevant haplotypes in the inferred potential haplotypes. The filtering of relevant haplotypes may comprise re-aligning reads against the different haplotypes and keeping haplotypes that the reads support. A read "supports" a haplotype when it aligns better against this haplotype than against others. The relevant haplotypes may be the haplotypes with the highest number of supporting reads. The method may enumerate two haplotypes. Indeed, the DNA may be diploid for instance in case of a human. Alternatively, the method may also enumerate more than two haplotypes. Indeed, the sequencing may provide several variants in a same region, and, when the variants are too far apart, it may be difficult to re-correct them because of the limited size of the reads and k-mers which may induce a loss of information. By enumerating two or more haplotypes, the variant-calling method therefore improves the recognition of variants.
After the enumerating of the two or more haplotypes, reads may be re-aligned to the haplotype(s) they support, and haplotypes may be aligned to the reference. Then, by a simple alignment composition, the method re-aligns reads against the reference, for example based on a known algorithm such as the algorithm of Smith-Waterman-Gotoh.

2.2 Variant Bayesian evaluation (filtering)

For each genomic position, the method may deduce potential variants out of the list of two or more haplotypes built during the haplotype reconstruction. Then, the method may perform a coarse grain filtering to detect candidate variants from the potential variants. By using base qualities and reads support information, the variant-calling method may implement a Bayesian framework, which performs a coarse-grained filtering. The variant-calling method may process data relative to each of the one or more sets independently. For instance, the method may process a set relative to somatic data based on the global Bayesian computing (as discussed in the following section). The variant-calling method may process the set relative to the somatic data by taking into account for germline variants and intercellular heterogeneity.

The one or more sets of reads comprises a first set of reads for germline variants and a second set of reads for somatic variants. The performing of the coarse grain filtering for each set is discussed in the following sections.

*2.2.1 Germline*

For a given genomic position, the variant-calling method evaluates the probability that any variant is more probable than the reference. The probability that any variant is more probable than the reference may be expressed based on the following formula:

$$\frac{P(var_1\ OR\ var_2\ OR\ ...\ var_n\ |pile)}{P(ref|pile)} = \frac{\sum_i P(var_i) \cdot P(pile|var_i)}{P(ref) \cdot P(pile|ref)}$$

wherein $var_i$ is the hypothesis that variant i is at the considered position. $P(var_i)$ is the prior probability of $var_i$ taking into account the homozygosity characteristics of the variant. $P(pile|var_i)$ is the probability that, knowing that there is a variant at a given position, this particular set of reads maps the given position. $P(pile|var_i)$ is computed by using the haplotype supporting reads information and the base qualities. $P(ref|pile)$ is a probability of errors. The method may consider if the variant is homozygous (same variant present on both pairs of chromosome) or heterozygous (variant present on a single chromosome of the pair). The method may compute a probability for each case. For instance, the homozygous variant case may be expressed based on the following formula:

$$P(pile|var_i) = \prod_{j\ \in S_{var_i}} (1 - e_j) \prod_{j\ \notin S_{var_i}} \frac{1}{3} e_j$$

with $S_{var_i}$ the set of reads that supports the variant *i*, and $e_j$ an error probability computed from the base quality of the read j at the position considered. The method may compute the error probability $e_j$ based on indicators provided by the sequencing technology. The method may neglect indel errors since they occurred to a much lower rate compared to substitutions.

*2.2.2 Somatic*

For the somatic case, the variant calling method may consider a set of hypothesis. The method performs an analysis on each genomic position of each candidate variant. The method considers germline variants and somatic variants on same genomic positions. The set comprises a first hypothesis that there is no germline variant and no somatic variant. The set comprises a second hypothesis that there is a germline variant and no somatic variant. The set comprises a third hypothesis that there is no germline variant and a somatic variant. The set comprises a fourth hypothesis that there is a germline variant and a somatic variant

Additionally, the method may take into account that the somatic variant might be present only in certain cells. The frequency of the presence of the somatic variant in cells is an unknown and is an additional hypothesis that the method may consider. For example, the third hypothesis with a somatic variant frequency *f* may be expressed based on the following formula:

$$P\big(pile_{soma}, pile_{germ}\big|var_{soma}(f), ref_{germ}\big) = P\big(pile_{soma}\big|var_{soma}(f), ref_{germ}\big) \cdot P\big(pile_{germ}\big| ref_{germ}\big)$$

where

$$P\big(pile_{soma}\big|var_{soma}(f), ref_{germ}\big) = \left( \prod_{i\ \in S_{var}^{soma}} f \cdot (1 - e_i) + (1-f) \cdot \frac{1}{3} e_i \right)\left( \prod_{i\ \notin S_{var}^{soma}} (f \cdot \frac{1}{3} e_i + (1-f) \cdot (1 - e_i) \right)$$

and

$$P\big(pile_{germ}\,|\,ref_{germ}\big) = \left( \prod_{i\,\in R^{germ}} (1 - e_i) \right) \cdot \left( \prod_{i\,\notin R^{germ}} \frac{1}{3} e_i \right)$$

## 2.3 Performance evaluation

The method may perform the coarse grain filtering for detecting the candidate variants. The coarse grain filtering ensures that very few true variant are left out even if the method retains too many candidates. The "recall" is a metric that tells how many variants the method has missed. The method may retrieve true variants from a dataset, for example a dataset provided by GIAB (acronym for Genome In A Bootle). The recall may be expressed with the formula *recall = TP/(TP + FN)* wherein TP represents true positives and FN represents true negatives. A value "0" of the recall may mean that the method has not selected any true variant whereas a value "1" of the recall may mean that the method has not forgotten any. The filtering is improved when the value of the recall is high while the number of selected variant candidates is low.

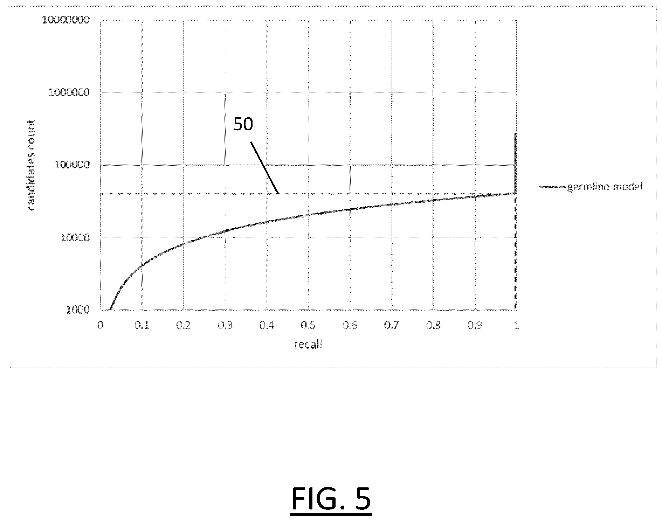

FIG. 5 illustrates an example of a germline candidate variants count as a function of recall for HG001 and chromosome 22. The method computes the graphs by varying a selection threshold, for example between an interval [-100 ; 100]. The method may comprise selecting a selection threshold based on the evolution of the recall. The dashed line 50 corresponds to the threshold selected by the method in this example. The recall equals 0.997 and the number of candidates is 44256 for the selected threshold in this example.

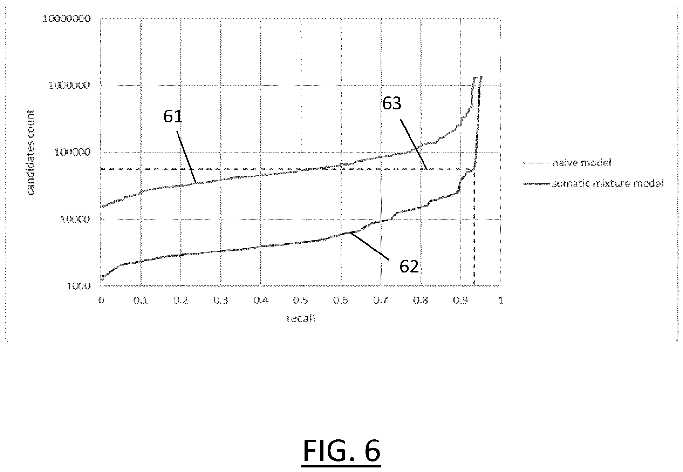

FIG. 6 illustrates an example of a somatic candidate variants count as a function of recall for COLO-829 and chromosome 22. As for the germline case, the graphs are obtained by varying a selection threshold and the method may select a selection threshold (the threshold may also variate in the interval [-100 ; 100]). The Naive model 61 is a germline Bayesian model applied independently to germline and somatic piles while the Somatic mixture model 62 is the Bayesian model combining both piles. The dashed line 63 corresponds to the threshold selected by the method (the recall equals 0.934 and the number of candidates is 56767).

## 2.4 Data pieces generation

Once the candidate variants have been determined, the variant calling method determines, for each detected candidate variant, one or more sets of data pieces each specifying a respective read aligned relative to the genomic position corresponding to the detected candidate variant. Each data piece comprises a 2D structure with a number of independent channels, for example higher than 3. Each data piece may also comprise a 1D structure and a set of scalars, as discussed in the following section. Each data piece specifies a respective read aligned relative to the genomic position, i.e., each data piece specifies one of the read of the pile of reads that map the given genomic position. The pile of reads may be illustrated in a pile-up image.

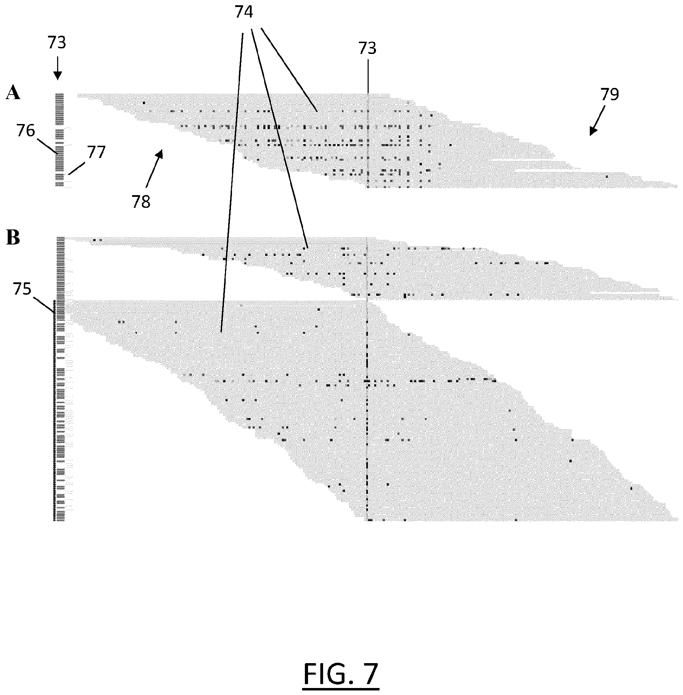

FIG. 7 illustrates an example of pile-up images. A pile-up image is a 2D representation of the reads intersecting a given genomic position (i.e., of reads that map to the given genomic position). Each line of the pile represents a respective read aligned on the genomic position 73. Each of the reads is aligned to the reference genome. Each of the reads has a respective sequence of bases that, when the read is aligned with the reference, comprises the genomic position 73. The reads may be arranged in the pile in order of appearance of the genomic position in their respective sequence.

Two different pile-up images are illustrated in FIG. 7. Image A is a pile-up image of a set of reads obtained for germline variants and image B is a pile-up image of two sets of reads, one obtained for germline variants and the other for somatic variants. The pile-up image represents two kind of information: read descriptors 73 and base descriptors 74. The read descriptors 73 are on the first left columns. The read descriptors include a somatic read descriptor 75, a descriptor of read supporting the reference 76 and a descriptor of read supporting the variant haplotype 77. Each read has a respective value for each read descriptor 75, 76 and 77, which is encoded in the data pieces. For instance, for the somatic read descriptor 75, each data piece comprises a true or false value depending on whether the read is for a germline or somatic variant.

The pile-up images also represents base descriptors 74. The base descriptors 74 are drawn on the read representations themselves in FIG. 7. The base descriptors 74 are encoded in the data piece of each read. For each read, the base descriptors 74 represent mismatches between the respective sequence of bases of the read and the reference genome. The base descriptors 74 may comprise a specific base descriptor for each potential mismatch, for example one for each of the bases A, T, G and C.

Examples of pile descriptors, read descriptors and base descriptors are now discussed. The method may implement all or any part of these descriptors, and the method may also optionally implement other descriptors. The method may encode the descriptors in the data piece specifying each read, for example on different channels. Each descriptor may be encoded on one or more independent channels depending on the complexity of the descriptor.

The pile descriptors (respectively the read descriptors or the base descriptors) each represent an aspect of the pile (respectively the read or the base) and consist of a value quantifying said aspect. In case the neural network is configured to take as input only one single set of data pieces, the set may correspond to a single pile of reads, and the pile descriptors may specify said pile. In case the neural network is configured to take as input several sets of data pieces, the one or more sets may correspond altogether to a same pile of reads, and the pile descriptors may specify said pile. Alternatively, each set may correspond to a respective different pile of reads or be seen as such, and the neural network may take as input pile descriptors specifying each such pile. The pile descriptors may be inputted to the one or more fully connected layers, for example aggregated with the output of each reduction layer (e.g. concatenated).

The pile descriptors may comprise a depth descriptor, which represents the depth of the pile (i.e., the number of reads that are stacked in the pile). The pile descriptors may consist of the number of reads in the pile for the considered pile. The method may encode the depth descriptor in one channel. The pile descriptors may also comprise a descriptor of Bayesian variant estimation (data from the prefiltering), for example encoded in four channels (e.g., p00, p01, p11 and p12 depending on the considered hypothesis).

The read descriptors may comprise a somatic descriptor, which may be present only for somatic data. The read descriptors may comprise a descriptor of support for haplotype, which describes the haplotype that the read supports (e.g., H0 when the read supports the reference, H1 when the read supports the first haplotype and H2 when the read supports the second haplotype). The descriptor of support for haplotype (also referred to as haplotype support descriptor) may be encoded in three channels (one for the reference genome and one for each of the two most supported haplotypes). Optionally, the read descriptors may comprise a mapping quality descriptor. The mapping quality consists of data provided by the aligner that performs the initial alignment. The mapping quality measures for each read a level of confidence with respect to alignment of the read. For instance, when a read is perfectly aligned in at least two regions of the genome, the mapping quality is null (i.e., has for example a zero "0" value). Optionally, the read descriptors may comprise a strandness descriptor which represents a sequenced strand and also a direction in which the sequencing is performed. The strandness may consist of data provided by the sequencing machine. Optionally, the read descriptors may comprise a descriptor of mate strandness. Indeed, depending on the sequencing technology, the reads may be provided in pairs. In that case, the mate corresponds to the other end of the pair, which is generally mapped a few thousand bases away.

The read descriptors have a one dimensional (1D) structure (one value for each read). The base descriptors have a two dimensional (2D) structure (one value for each base of each read). The base descriptors comprise a base type descriptor, which defines the type of the base (i.e., A, T, C or G). The base descriptors may be encoded on four channels (one for each type of base). The base descriptors may comprise a base quality descriptor, which describes the quality of identification of the base. The base descriptors may comprise a reference base descriptor, which is the corresponding base in the reference genome. The base descriptors may comprise a mismatch descriptor, which describes the difference between the base and the corresponding base in the reference genome (e.g., based on a Boolean value). The base descriptors may comprise a descriptor of insertion size and, alternatively or additionally, a descriptor of deletion size, which may be computed by the method by comparing the alignment of each read on the reference (the alignment comprising insertions and deletions).

The length of each data piece may be constant and the data pieces may be all centered on the genomic position. Each data piece includes the sequence of base descriptors corresponding to the read and an additional start sequence 78 and an additional end sequence 79 positioned respectively at the beginning and at the end of the sequence of base descriptors corresponding to the read (shown as blank on the figure). The size of the additional start and end sequences variate to align the respective sequence of each read on the reference genome. The additional start or end sequences consist of a sequence of one or more base descriptors having null values. The length corresponds to the size of two reads (in case of constant-length reads, or about twice the longest read otherwise), for example 300 bases. The method may also truncate the data, and use a lower length (lower than 300 bases). The constant length facilitates processing, for example via convolutions.

**Section 3: Variant Filtering**

3.1 Deep learning model

Unlike many Machine Learning Approaches based on state-of-the arts image classifications models that use Convolutional Neural Networks (CNN) with millions of parameters and that are not specific to genomic data, the method may implement an architecture of the neural network that takes into account the specificities of the genomic data. This allows reducing the size of the model and/or increasing the accuracy.

The reads are 1D structures and their representation in piles is arbitrary. Indeed, the y coordinate (i.e., in the direction of the pile in a pile-up image) is not informative. In the method, the structure of the neural network comprises operators that are order independent (such as sum, average, minimum, maximum for instance). For that reason, the neural network of the method is an improved solution for variant calling relative to prior-art methods that are based on standard 2D CNN. The neural network has an optimized architecture that reduces the computational cost and demonstrates high performance.

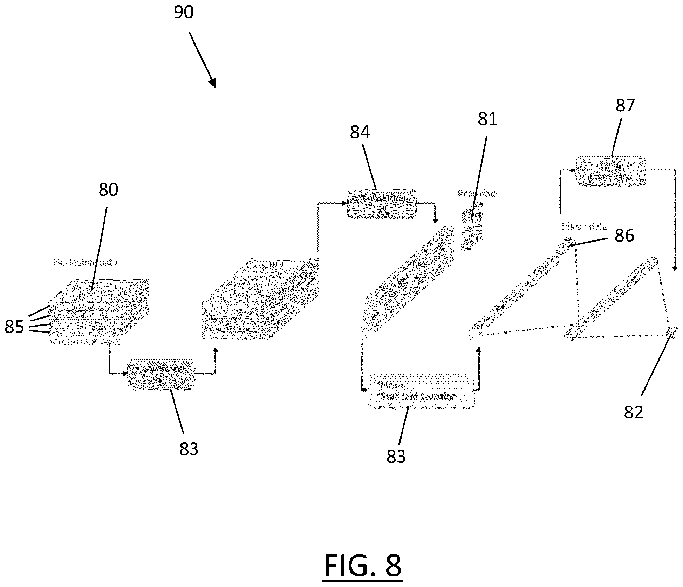

FIG. 8 illustrates an example of architecture of the neural network for calling germline variants. The neural network for the germline case consists of a symmetric function 90 configured to take as input and process a set of read-specifying data pieces 80-81, as well as pile descriptors 86. Each data piece 85 specifies a respective read aligned relative to a genomic position and includes the sequence of base descriptors of the read (each base descriptor is encoded on one or more channels). Each data piece 85 comprises a 2D structure (i.e., one direction for the sequence of bases and one direction for the corresponding base descriptors associated with each base of the sequence). The reads are superposed to form a pile, which provides to the set of data pieces a 3D structure 80 (one direction for the reads, one direction for the sequence of bases of each read and one direction for the base descriptors). The neural network is configured to output information 82 with respect to presence of a variant at the genomic position.

The function 90 for the germline case comprises several convolutional layers 83, 84 for processing each respective sequence of base descriptors. Each convolutional layer applies several one-dimensional convolutional filters. The function 90 comprises a first 1 x 1 convolution layer 83 for re-encoding the base descriptors in a predetermined number of channels, for example five channels. The first convolution layer 83 transforms information at base level in a uniform and nonlinear way. The first convolution 83 allows normalizing input data. Then, the function 90 comprises a second $l$ x 1 convolution layer 84 for reducing the data piece of each read from $l$ to 1 position, wherein $l$ is the size of each data piece. The second convolution 84 outputs a predetermined number of channels, for example ten channels. The function 90 comprises a concatenation of read descriptors 81 for each read with the output of the second convolution. The function 90 inputs the concatenation of the read descriptors and the output of the second convolution in a first reduction layer. The reduction layer collapses order-independently the output of the second convolutional layer concatenated with the read descriptors into a set of features based on order-independent operators, such as mean and standard deviation. The reduction layer collapses the n reads to one pack of a set of features (for example twenty features). The function 90 aggregates the pile descriptors 86 to the pack of the set of features. The set corresponds to a single pile of reads, and the pile descriptors specify said pile. Then, the function 90 comprises several fully connected layers 87 configured for processing the output of the reduction layer and performing classification.

The neural network may have 5240 trainable parameters. The machine-learning method may machine-learn the neural network based on a set of hyper-parameters, which includes a learning rate (e.g., 1e-3), a number of iterations (e.g., 100), a percent of examples for inner validation (e.g., 10 %), an optimization algorithm (e.g., ADAM), an activation function (e.g., Mish) and a cost function (e.g., a weighted cross entropy).

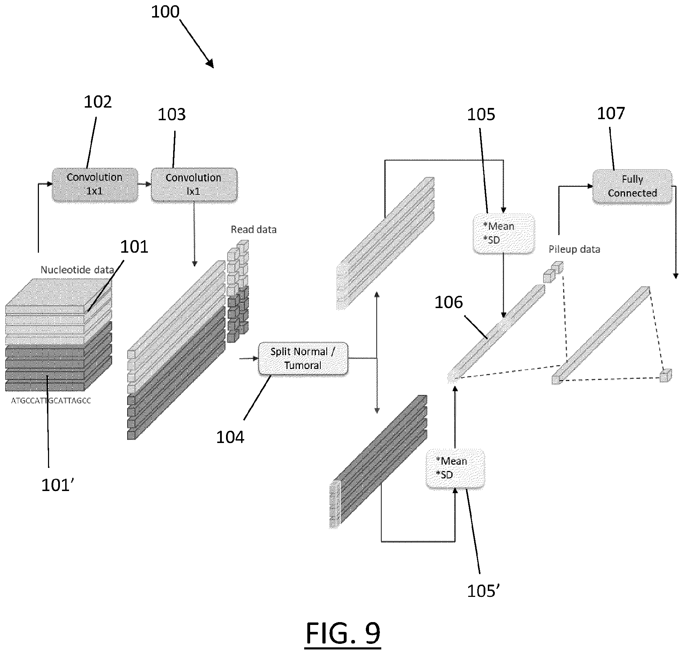

FIG. 9 illustrates an example of architecture of the neural network 100 for calling somatic variants. The neural network 100 takes as input two sets of reads: a first set 101 for germline variants (i.e. reads from healthy cells of an individual, e.g. a human patient having a cancer disease), and a second set 101' for somatic variants (i.e. reads from cancer cells of the same individual). The neural network 100 comprises a first symmetric function for processing the first set and a second symmetric function for processing the second set. The first function consists of sub-architectures 102, 103, 104 and 105 and the second function consists of sub-architectures 102, 103, 104 and 105'. The first and second functions are the same, i.e. comprise the same parameters values and shared weights. Each of the first and the second functions comprises two convolutional layers 102, 103 for processing each sequence of base descriptors. The convolution layers (the first 1 x 1 convolution layer 102 and the second $l$ x 1 convolution layer 103) are the same for the first and second functions (i.e. comprise shared weights for the first and second functions). The neural network 100 concatenates read descriptors with the output of the second convolution layer.

After the concatenation, the neural network 100 comprises a split 104 of the output of the second convolution layer concatenated with the respective read descriptors to separate the output corresponding to the first set and the output corresponding to the second set. After the splitting, each of the first and second functions comprises a respective reduction layer 105, 105' for processing the output corresponding to each set independently (e.g., successively or in parallel). Each of the respective reduction layers 105, 105' collapses order-independently the outputs of the second convolutional layer into a respective set of features based on order-independent operators. The sets of features are the outputs of the first and second functions. The reduction layers 105, 105' may be the same, i.e. may comprise same order-independent operators.

Then, the neural network 100 comprises a layer for aggregating the outputs of the first and second functions, i.e. the set of features corresponding to the first set and the set of features corresponding to the second set. For instance, the aggregation layer may concatenate the two sets of features, thereby forming a single set of features 106. The aggregation layer may optionally aggregate pile descriptors with the output of each reduction layer (e.g. concatenate the pile descriptors with the single set of features 106). The two sets may correspond altogether to a same pile of reads, and the pile descriptors specify said pile.

After the aggregation layer, the neural network 100 comprises one or several fully connected layers 107 configured for performing classification. The several fully connected layers 107 take as input the output of the aggregation layer (i.e. the concatenation of the output of each reduction layer and, optionally, of the pile descriptors). A difference with the fully connected layers discussed in the germline case may be the size of the input, which may be twice as large in the somatic case. The machine-learning method may machine-learn the neural network for somatic variants, for example based on 9735 trainable parameters.

3.2 Performance

*3.2.1 Metrics*

Performance of the neural network is now presented based on several metrics. The metrics measure how good the positive class is predicted. Indeed, the negative class is vastly majoritarian since 99.9% of positions are not mutated (99.9 % of positions in the genome do not comprise a variant). In this context, other metrics are not informative since they give 0.999 with a model predicting always the negative class.

In the following, TP refers to True Positives, FP to False Positives and FN to False Negatives. A first metric is precision, which is formulated as TP / (TP + FP). Precision measures how good a neural network for variant calling is at predicting positives that are not false positives. A second metric is recall, which is formulated as TP / (TP + FN). Recall measures how good the neural network for variant calling is at non forgetting any true variant. A third metric is F1-Score, which is formulated as 2*Precision*Recall / (Precision + Recall). The F1-Score is a geometric mean of the precision and the recall. A fourth metric is number of errors. At genome scale, the F1-score might be so close to 1.0 that it is difficult to differentiate different model performance. In that situation, one might use the number of mispredictions (i.e., the number of errors) to differentiate properly the performance.

*3.2.2 Datasets and results*

In the following, the neural networks are trained based on publicly available ground truth datasets. The results of the neural network are compared with a list of prior-art variant callers. The list of prior-art variant callers includes Gatk4, Deepvariant 0.9 and the CNN classifier EfficientNet.

The results for the germline case are now discussed. For the germline case, the neural network and each prior-art variant callers are trained based on a training set, which is the individual genome HG001. The individual genome HG001 is an individual genome cross validated by multiple technologies by the Genome In A Bottle consortium (GIAB consortium). The performance of the neural network and each prior-art variant callers are computed based on a validation set, which is the individual genome HG002. The performance is appreciated based on the previously introduced metrics.

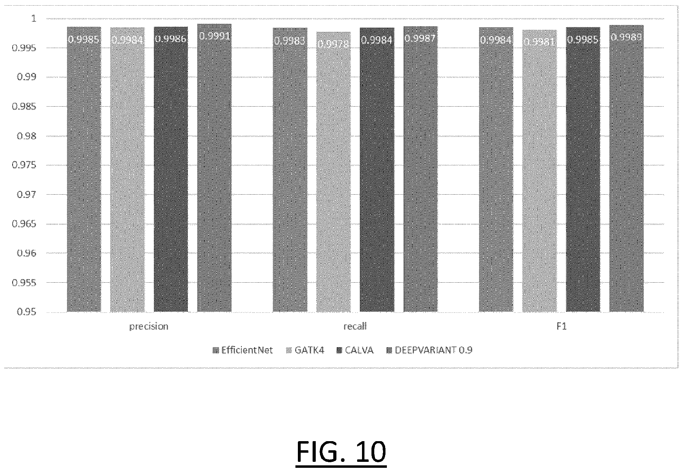

FIG. 10 illustrates an example of results for the precision, the recall and the F1-scores metrics for the germline case. F1-scores of the neural network and each of the prior-art variant callers are very close one to another.

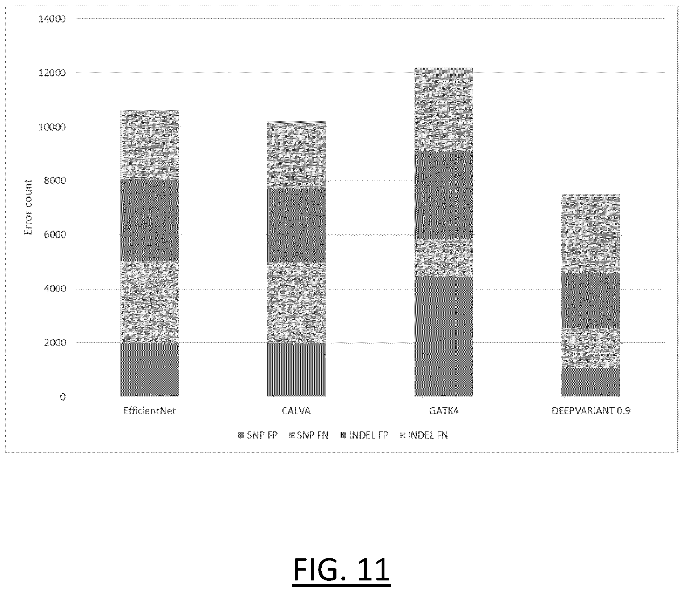

FIG. 11 illustrates an example of results for the number of errors metric for the germline case. The neural network has performance close to Deepvariant 0.9 but has only about 4000 parameters where Deepvariant 0.9 works with 24 M of parameters. Moreover, Deepvariant 0.9 has been trained on 300 M examples while the neural network has been trained on only 1 M examples. Indeed, Deepvariant 0.9 presents inferior performance when trained on the same dataset comprising 1 M examples. Furthermore, Deepvariant 0.9 does not manage the somatic case.

The results for the somatic case are now discussed. For the somatic case, the results are computed based on publicly available somatic reference standard. The results comprise results from COLO829 cancer cell line with the blood cell line from the same donor. The consensus is obtained after whole genome sequencing on four independent Illumina platforms and using multiple variant callers. The training set comprises 70000 examples.

The results also comprise results from HCC1143 cancer cell line somatic variants consensus obtained on two independent Illumina platforms and processed by prior-art Mutect2, Strelka2 and Lancet variant callers.

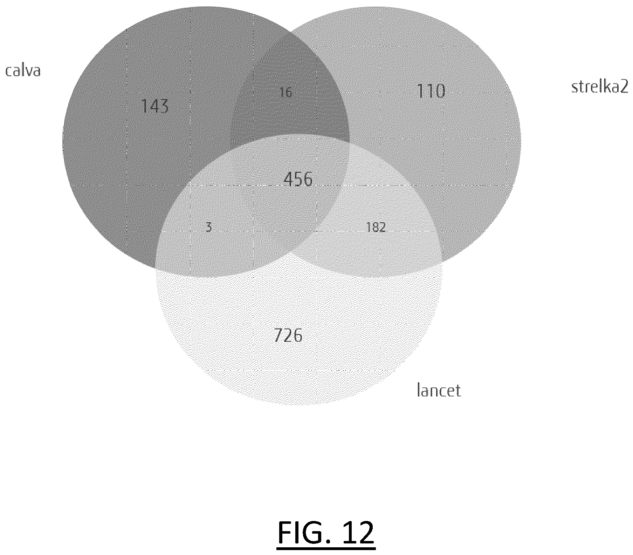

FIG. 12 illustrates an example of a comparison of results for the somatic case between the neural network and the prior-art variant callers Strelka2 and Lancet. The somatic sequencing is especially challenging and made difficult by the absence of a consensus ground truth of variants. This is even more problematic for learning based approaches. Nevertheless, the results are based on approximations of ground truth somatic datasets from works trying to establish a consensus. The results show that the neural network compares favorably to the two state of the art variant callers Lancet and Strelka2.

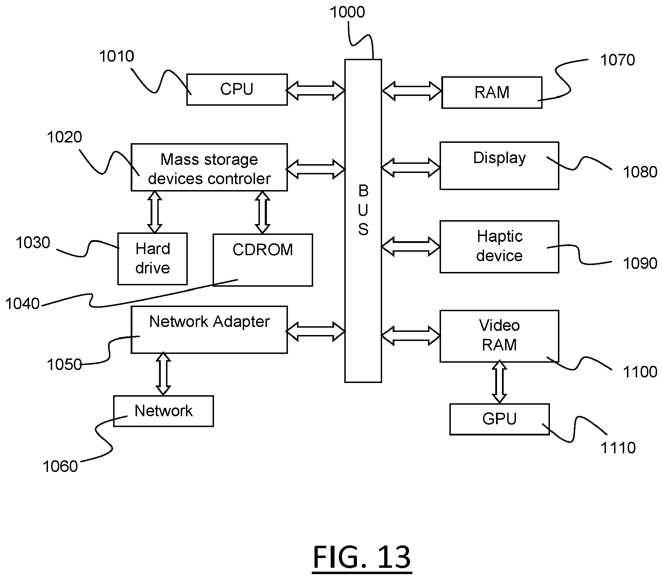

FIG. 13 shows an example of the system, wherein the system is a client computer system, e.g. a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks; and CD-ROM disks 1040. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method.

## Claims

1. A computer-implemented method for machine-learning a neural network for variant calling with respect to a reference genome, the neural network being configured to take as input one or more sets of data pieces each specifying a respective read aligned relative to a genomic position of the reference genome, and to output information with respect to presence of a variant at the genomic position, the neural network comprising, for each set of data pieces, a respective function configured to take as input and process the set of data pieces, the respective function being symmetric.

2. The machine-learning method of claim 1, wherein each data piece includes a respective sequence of base descriptors of the respective read, and each respective function comprises one or more convolutional layers for processing each respective sequence of base descriptors, each convolutional layer applying one or more one-dimensional convolutional filters.

3. The machine-learning method of claim 2, wherein the base descriptors comprise one or more descriptors representing insertion size and/or deletion size, for example an insertion size descriptor and a deletion size descriptor.

4. The machine-learning method of claim 2 or 3, wherein each respective function comprises a reduction layer for collapsing order-independently the output of the one or more convolutional layers into a set of features.

5. The machine-learning method of claim 4, wherein the reduction layer further takes as input read descriptors for each respective read, wherein optionally the read descriptors comprise a haplotype support descriptor.

6. The machine-learning method of claim 5, wherein the reduction layer comprises one or more order-independent operators, such as a mean and/or a standard deviation.

7. The machine-learning method of claim 5 or 6, wherein the neural network comprises one or more fully connected layers configured for processing the output of each reduction layer and performing classification.

8. The machine-learning method of claim 7, wherein the neural network further takes as input pile descriptors, wherein optionally the pile descriptors comprise a descriptor representing depth and/or a descriptor representing a Bayesian variant estimation.

9. The machine-learning method of any one of claims 1 to 8, wherein the one or more sets of reads comprise a first set of reads for germline variants and a second set of reads for somatic variants, the neural network comprising:

- a first function for the first set of reads and a second function for the second sets of reads, and
- a layer for aggregating the output of the first and second functions.

10. A computer-implemented method for variant calling with respect to a reference genome, the variant-calling method comprising:

- providing (S700), as input, one or more sets of data pieces each specifying a respective read aligned relative to a genomic position of the reference genome;
- applying (S800), to the input, a neural network machine-learnt according to any one of claims 1 to 9, so as to output information with respect to presence of a variant at the genomic position, the application of the neural network comprising applying, for each set of data pieces, the respective symmetric function of the neural network configured to take as input and process the set of data pieces.

11. A computer-implemented method for variant calling with respect to a reference genome, the variant-calling method comprising:

- providing one or more sets of reads aligned relative to the reference genome;
- determining a set of regions of interest in the reference genome by comparing the one or more sets of reads with the reference genome; and
- for each given region of interest:

-- performing a haplotype reconstruction based on the provided one or more sets of reads of the given region to determine two or more haplotypes;
-- re-aligning the one or more sets of reads of the given region based on the two or more haplotypes;
-- deducing potential variants of the given region based on the re-aligned one or more sets of reads and the two or more haplotypes;
-- performing a coarse grain filtering to detect candidate variants from the potential variants, each detected candidate variant corresponding to a respective genomic position; and
-- for each detected candidate variant:

--- determining one or more sets of data pieces each specifying a respective read aligned relative to the genomic position corresponding to the detected candidate variant; and
--- performing the providing (S700) and the applying (S800) of the computer-implemented method of claim 10 with the one or more sets of data pieces.

12. The computer-implemented method of claim 11, wherein the performing of the haplotype reconstruction comprises:

- inferring a set of potential haplotypes by enumerating a predetermined number of longest paths in a directed acyclic graph; and optionally
- selecting a subset of haplotypes from the set of potential haplotypes, the subset of haplotypes being potential haplotypes of the set having the highest number of supporting reads, the subset of haplotypes corresponding to the two or more haplotypes that the haplotype reconstruction determines.

13. The computer-implemented method of claim 11 or 12, wherein the one or more sets of reads comprises a first set of reads for germline variants, the deducing of potential variants of the given region comprising, for germline variants, evaluating a probability that a variant is more probable than the reference, wherein optionally the one or more sets of reads comprises a second set of reads for somatic variants, the deducing of potential variants of the given region considering, for somatic variants:

- a presence of a germline variant and/or the presence of a somatic variant, and
- a somatic variant frequency.

14. A data structure comprising a neural network machine-learnt according to any one of claims 1 to 9, a computer program comprising instructions for performing the machine-learning method of any one of claims 1 to 9, and/or a computer program comprising instructions for performing the variant-calling method of any one of claims 10 to 13.

**15.** A device comprising a data storage medium having recorded thereon the data structure of claim 14.

Providing, as input, one or more sets of data pieces each specifying a respective read aligned relative to a genomic position of the reference genome — S700

Applying, to the input, a neural network machine-learnt according to a machine-learning method, so as to output information with respect to presence of a variant at the genomic position, the application of the neural network comprising applying, for each set of data pieces, the respective symmetric function of the neural network configured to take as input and process the set of data pieces — S800

## FIG. 1

Providing one or more sets of reads aligned relative to the reference genome — S000

Determining a set of regions of interest in the reference genome by comparing the one or more sets of reads with the reference genome — S100

For each given region of interest of the set:

Performing a haplotype reconstruction based on the provided one or more sets of reads of the given region to determine two or more haplotypes — S200

Re-aligning the one or more sets of reads based on the two or more haplotypes — S300

Deducing potential variants based on the re-aligned one or more sets of reads and the two or more haplotypes — S400

Performing a coarse grain filtering to detect candidate variants from the potential variants, each detected candidate variant corresponding to a respective genomic position — S500

For each detected candidate variant :

Determining one or more sets of data pieces each specifying a respective read aligned relative to the genomic position corresponding to the detected candidate variant — S600

Performing the providing S700 and the applying S800 of FIG. 1 with the one or more sets of data pieces of the first variant-calling method

## FIG. 2

FIG. 3

FIG. 4

## FIG. 5

## FIG. 6

FIG. 7

FIG. 8

FIG. 9

FIG. 10

# FIG. 11

# FIG. 12

1000

1010

CPU

1070

RAM

1020

Mass storage
devices controler

Display

1080

B
U
S

1030

Hard
drive

CDROM

Haptic
device

1090

1040

Network Adapter

Video
RAM

1050

1100

Network

GPU

1060

1110

## FIG. 13

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/065847 A1 (VALOUEV ANTON [US] ET AL) 4 March 2021 (2021-03-04)<br>* claims 14,18,25 *<br>* paragraph [0010] – paragraph [0011] *<br>* paragraph [0030] – paragraph [0031] *<br>* paragraph [0033] *<br>* paragraph [0055] – paragraph [0058] *<br>* paragraph [0062] *<br>* paragraph [0064] *<br>* paragraph [0073] – paragraph [0074] *<br>* paragraph [0083] *<br>* paragraph [0092] *<br>* paragraph [0142] *<br>* paragraph [0185] – paragraph [0186] *<br>* paragraph [0257] – paragraph [0258] *<br>* paragraph [0290] *<br>* paragraph [0300] *<br>* paragraph [0308] *<br>----- | 1-15 | INV.<br>G06N20/00<br>G16B20/20<br>G16B30/10 |
| X | US 2021/098078 A1 (LOZAC'HMEUR ARIANE [US] ET AL) 1 April 2021 (2021-04-01)<br>* paragraph [0090] – paragraph [0091] *<br>* paragraph [0102] *<br>* paragraph [0114] *<br>* paragraph [0121] *<br>* paragraph [0129] *<br>* paragraph [0168] – paragraph [0169] *<br>* paragraph [0173] – paragraph [0174] *<br>* paragraph [0200] – paragraph [0203] *<br>* paragraph [0207] – paragraph [0208] *<br>* paragraph [0320] – paragraph [0321] *<br>* paragraph [0593] – paragraph [0594] *<br>* paragraph [0612] *<br>----- | 1-15 | **TECHNICAL FIELDS SEARCHED (IPC)**<br><br>G06N<br>G06F<br>G16B |

-/--

The present search report has been drawn up for all claims

| Place of search | Date of completion of the search | Examiner |
|---|---|---|
| Munich | 8 February 2022 | Martínez Cebollada |

EPO FORM 1503 03.82 (P04C01)

Europäisches
Patentamt

European
Patent Office

Office européen
des brevets

## EUROPEAN SEARCH REPORT

Application Number

EP 21 30 6133

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/104297 A1 (VENN OLIVER CLAUDE [US] ET AL) 8 April 2021 (2021-04-08)<br>* paragraph [0018] - paragraph [0019] *<br>* paragraph [0039] *<br>* paragraph [0097] - paragraph [0098] *<br>* paragraph [0127] - paragraph [0129] *<br>* paragraph [0136] *<br>* paragraph [0166] *<br>* paragraph [0251] *<br>* paragraph [0254] *<br>* paragraph [0292] *<br>* Facsimile page 22 *<br>* paragraph [0320] *<br>* paragraph [0324] - paragraph [0325] *<br>----- | 1-15 | |
| X | US 2021/257050 A1 (LAM HUGO Y K [US] ET AL) 19 August 2021 (2021-08-19)<br>* paragraph [0003] *<br>* paragraph [0006] - paragraph [0012] *<br>* paragraph [0016] - paragraph [0017] *<br>* paragraph [0020] *<br>* paragraph [0024] - paragraph [0026] *<br>* paragraph [0038] - paragraph [0039] *<br>* paragraph [0058] *<br>* paragraph [0079] - paragraph [0083] *<br>* paragraph [0130] *<br>* paragraph [0134] *<br>* paragraph [0136] - paragraph [0138] *<br>* paragraph [0150] - paragraph [0154] *<br>* paragraph [0158] - paragraph [0160] *<br>* paragraph [0192] - paragraph [0194] *<br>* paragraph [0235] *<br>* paragraph [0262] - paragraph [0263] *<br>* Facsimile page 3 to page 4 *<br>* Facsimile page 6 *<br>* Facsimile page 8 *<br>----- | 1-15 | TECHNICAL FIELDS SEARCHED (IPC) |

The present search report has been drawn up for all claims

| Place of search | Date of completion of the search | Examiner |
|---|---|---|
| Munich | 8 February 2022 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 30 6133

This annex lists the patent family members relating to the patent documents cited in the above-mentioned European search report.
The members are as contained in the European Patent Office EDP file on
The European Patent Office is in no way liable for these particulars which are merely given for the purpose of information.

08-02-2022

| Patent document cited in search report | | Publication date | Patent family member(s) | | Publication date |
|---|---|---|---|---|---|
| US 2021065847 | A1 | 04-03-2021 | US | 2021065847 A1 | 04-03-2021 |
| | | | WO | 2021041840 A1 | 04-03-2021 |
| US 2021098078 | A1 | 01-04-2021 | US | 2021098078 A1 | 01-04-2021 |
| | | | WO | 2021022225 A1 | 04-02-2021 |
| US 2021104297 | A1 | 08-04-2021 | CN | 112218957 A | 12-01-2021 |
| | | | EP | 3781709 A1 | 24-02-2021 |
| | | | US | 2021104297 A1 | 08-04-2021 |
| | | | WO | 2019204360 A1 | 24-10-2019 |
| US 2021257050 | A1 | 19-08-2021 | CN | 113168886 A | 23-07-2021 |
| | | | EP | 3837690 A1 | 23-06-2021 |
| | | | JP | 2021534492 A | 09-12-2021 |
| | | | US | 2021257050 A1 | 19-08-2021 |
| | | | WO | 2020035446 A1 | 20-02-2020 |

EPO FORM P0459

For more details about this annex : see Official Journal of the European Patent Office, No. 12/82